(19) Europäisches Patentamt
European Patent Office
Office européen des brevets

(11) Publication number: 0 232 089 B1

# (12) EUROPEAN PATENT SPECIFICATION

(45) Date of publication of patent specification: 13.03.91 Bulletin 91/11

(51) Int. Cl.[5]: C07C 235/84, A61K 31/165

(21) Application number: 87300580.5

(22) Date of filing: 23.01.87

(54) **Quinone amides, their production and use.**

(30) Priority: 28.01.86 JP 16182/86

(43) Date of publication of application: 12.08.87 Bulletin 87/33

(45) Publication of the grant of the patent: 13.03.91 Bulletin 91/11

(84) Designated Contracting States: AT BE CH DE ES FR GB GR IT LI LU NL SE

(56) References cited:
EP-A- 0 025 692
EP-A- 0 092 136
EP-A- 0 171 251

(73) Proprietor: TAKEDA CHEMICAL INDUSTRIES, LTD.
3-6, Doshomachi 2-chome Chuo-ku
Osaka (JP)

(72) Inventor: Terao, Shinji
26-3,Shinsenri-minamimachi 2-chome
Toyonaka Osaka 565 (JP)
Inventor: Maki, Yoshitaka
5-17, Oharano-kamisatotorimicho
Nishikyo-ku Kyoto 610-11 (JP)

(74) Representative: Laredo, Jack Joseph et al
Elkington and Fife Beacon House 113
Kingsway
London, WC2B 6PP (GB)

EP 0 232 089 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel quinone derivatives which possess therapeutic and prophylactic actions against bronchial asthma, immediate-type allergy, various types of inflammations, arteriosclerosis, endotoxin shock associated with bacterial infection, etc., to a method of preparing the same and to pharmaceutical compositions containing the same, and can be utilized in the field of medicine.

Heretofore, it has been regarded as difficult to perform effectively the treatment or prevention of bronchial asthma. In recent years, SRS-A (slow-reacting substance of anaphylaxis) which has long been known as one of the important mediators for immediate-type hypersensitivity and asthma was shown to be composed of the 5-lipoxygenase metabolites of arachidonic acid, namely leukotrienes, which have been attracting attention. The leukotrienes are a potent chemical mediator for the allergic or inflammatory reaction, and is considered to cause mainly constriction of peripheral airways in the lung, being related with respiration distress accompanied by brochial asthma. Also, the leukotrienes possess the capabilities to enhance the capillary permeability and to strongly produced chemotactic activity of leukocytes, and are intimately associated with edema and cellular infiltration, which are typical symptoms of inflammation. They, furthermore,with their potent vasoconstriction action, are considered to contribute eventually to the incidence of coronary insufficiency and angina pectoris. Accordingly as the pathophysiologic significance of the leukotrienes has become clarified as is described above, the importance of the 5-lipoxygenase, a key enzyme for the biosynthesis of the leukotrienes, has come to be recognized.

As the compound exhibiting 5-lipoxygenase inhibitory activity, there have already been known flavone compounds, quinone compounds [the U.S. Pat. No.4271083, EPC Laid-Open No. 21841 and U.S. Pat. No. 4358461], catechol compounds [Clin. Exp. Pharmacol. Physiol., 8, 654-655 (1981)], 116, 612-618 (1983)], acetylene compounds [Eur. J. Biochem., 139, 577-583 (1984), etc., but all of them are far from being adequately satisfactory in terms of drug metabolism and bioavailability dynamics.

This invention provides novel quinone compounds which undergo less inactivation due to the metabolic system and exhibit more long-acting efficacy with a lower dosage than the known compounds shown to possess 5-lipoxygenase inhibitory activity.

This invention is concerned with :

1. A compound of the general formula :

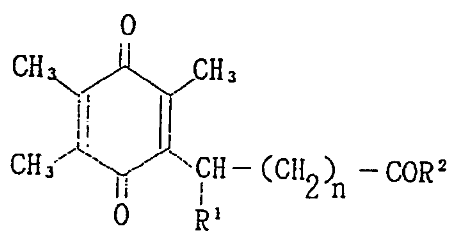

(I)

wherein

$R^1$ is phenyl unsubstituted or a phenyl substituted at an optional position on the ring with
- hydroxyl,
- halogen,
- alkyl having 1 to 3 carbon atoms or
- alkoxy having 1 to 3 carbon atoms,

$R^2$ is amino unsubstituted, an amino substituted with
- $(C_{1-4})$alkyl
- $(C_{6-10})$aryl unsubstituted,
- $(C_{6-10})$aryl substituted with
  - hydroxyl,
  - amino,
  - nitro,
  - halogen,
  - methyl or
  - methoxy,
- benzyl,
- α-phenethyl,
- β-phenethyl,

1-(α-naphthyl)ethyl,
amino acid residue resulting from eliminating one hydrogen from the amino the group consisting of
glycine,
arginine,
histidine,
aspartic acid,
proline,
phenylalanine,
methionine,
alanine,
leucine and
glutamic acid the amino acid residue being unesterified or esterified at the carboxyl group with benzyl alcohol or
the amino acid residue being unprotected or protected, when the amino acid residue has an amino group, with
nitro
cyclic amino selected from a group consisting of
morpholino,
piperidino,
piperazino or
pyrrolidino,
the cyclic amino being unsubstituted or substituted with
methyl,
ethyl,
ProPyl,
pyrrolidinocarbonylmethyl,
4-fluorophenylcarbonylpropyl,
phenyl,
naphthyl,
2-methoxyphenyl,
4-methylphenyl,
4-bromophenyl,
benzyl,
2-phenylethyl,
4-fluorophenylmethyl,
4-methoxyphenylmethyl,
3,4,5-trimethoxyphenylmethyl,
diphenylmethyl,
aminocarbonyl,
6-(9-β-D-ribofuranoside)-adenyl or
4-amino-6,7-dimethoxy-quinazolinyl,
2-furylcarbonyl and n is an integer of 3 to 10, or a hydroquinone derivative thereof ;

2. a method of preparing a compound representable by the general formula (I), which comprises allowing an activator of carboxylic acid to react with a compound representable by the general formula :

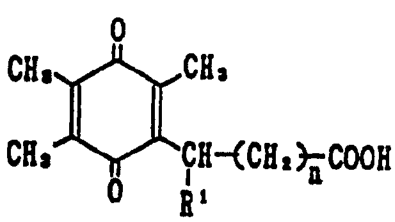

(II)

(wherein each symbol is of the same meaning as defined above) to lead to a reactive derivative at the carboxyl group, then by allowing this compound to react with a compound representable by the general formula ; $R^2$-H (III) (wherein $R^2$ is of the same meaning as defined above), and

3. a pharmaceutical composition, which comprises as an effective component a quinone derivative representable by the general formula (I) or a hydroquinone derivative thereof.

In the above general formula (I), the substituted phenyl group represented by $R^1$ may have 1~5, preferably 1~3, most preferably 1 substituent(s) at an optional position on the ring, and these substituents are exemplified by a hydroxyl group, halogen atom such as fluorine, chlorine, bromine, etc., alkyl groups having 1~3 carbon atoms such as methyl, ethyl, etc., alkoxy groups having 1~3 carbon atoms such as methoxy, ethoxy, etc., and, among them, fluorine, methyl and methoxy are prefarable. It is preferable that the sustituent is sustituted at a meta or para position on the phenyl ring. $R^1$ is preferably an unsubstituted phenyl or a phenyl having a halogen at its meta or paraposition.

The optionally substituted amino group represented by $R^2$ includes an unsubstituted amino group, substituted amino and cyclic amino. Substituent groups of the substituted amino group are exemplified by an alkyl having 1~4 carbon atoms such as methyl, ethyl, propyl, butyl, etc., an aryl having 6~10 carbon atoms such as phenyl, naphthyl, etc. (these aryl groups may have further substituents such as hydroxyl, amino, nitro, halogen, methyl, methoxy, etc. at an optional position on the ring), etc. Practical embodiments of the substituted amino group are exemplified by a monoor di-alkyl amino having 2~4 carbon atoms (methylamino, ethylamino, isopropylamino, dimethylamino), aralkylamino (benzylamino, α-phenethylamino, β-phenethylamino, 1-(α-naphthyl)ethylamino], phenylamino, substituted phenylamino (p-hydroxyphenylamino, p-methoxyphenylamino, m-chlorophenyl-amino), diphenylamino, or amino acid residue resulting from eliminating one hydrogen from the amino group of an amino acid (glycine residue, arginine residue, histidine residue, aspartic acid residue, proline residue, phenylalanine residue, methionine residue, alanine residue, leucine residue). The cyclic amino is exemplified by morpholino, piperidino, piperazino, pyrrolidino, and these may have 1~3 substituents at an optional position of the ring. These substituents are exemplified by optionally substituted alkyl (methyl, ethyl, propyl, pyrrolidinocarbonyl methyl, 4-fluorophenyl-carbonyl propyl, etc.), optionally substituted aryl (phenyl, naphthyl, 2-methoxyphenyl, 4-methylphenyl, 4-bromophenyl, etc.), optionally substituted aralkyl (benzyl, 2-phenyl-ethyl, 4-fluorophenyl methyl, 4-methoxyphenyl methyl, 3,4,5-trimethoxyphenyl methyl, diphenyl methyl, etc.), aminocarbonyl, 6-(9-β-D-ribofuranoside)adenyl, 4-amino-6,7-dimethoxy-quinazolinyl, etc. As $R^1$, amino, carboxymethylamino and 4-phenethylpiperazino are preferable, and amino is the most preferable.

n is preferably 4, 5 or 6. Among the compounds (I), a compound of formula (I) wherein $R^1$ is phenyl or m- or p-halogenophenyl, $R^2$ is amino, carboxymethylamino or 4-phenethylpiperazino and n is 4, 5 or 6, is the most preferable.

A compound representable by the general formula (I) can be prepared by allowing an activator of carboxylic acid to react with a compound (II) to lead to a reactive derivative at the carboxyl group, then by allowing this compound to react with a compound (III).

In the reaction of a compound (II) with an activator of carboxylic acid, the activator of carboxylic acid is exemplified by thionyl chloride, phosphorus pentachloride, chlorocarbonic ester (methyl chlorocarbonate, ethyl chlorocarbonate), dicyclocarbodiimide (DCC), etc., and dicyclocarbodiimide, and p-nitrophenol or hydroxysuccinic imide may be used together. This reaction is usually conducted in the presence of, for example, halogenated hydrocarbons such as methylene chloride, chloroform etc., ethers such as tetrahydrofuran, dioxane, dimethyl ether, diethyl ether, isopropyl ether, etc., dimethyl formamide, etc.. The reaction temperature usually ranges from – 10°C to 50°C.

In this reaction, when thionyl chloride or phosphorus pentachloride is employed as an activator of carboxylic acid, an acid halide is obtained as the resultant reactive derivative ; when chlorocarbornate is employed as an activator of carbxylic acid, an acid anhydride mixture is obtained as the reactive derivative ; and when carbodiimide is employed as a carboxylic acid activator, an active ester is obtained as the resultant reactive derivative.

The reaction between a reactive derivative at the carboxyl of a compound (II) and an amino compound, when the reactive derivative is an acid halide, is conducted in, for example, an anhydrous solvent such as dichloromethane, triethylamine, etc. or a water-containing solvent such as water-containing acetone, water-containing tetrahydrofuran, etc. in the presence of a deacidifying agent (pyridine, triethylamine, potassium carbonate, sodium carbonate, etc.). The reaction temperature ranges from about – 10°C to about 10°C. When the reactive derivative is an active ester or an acid anhydride mixture, the reaction can be conducted in a solvent similar to that employed for the reaction of a compound (II) with a carboxylic acid activator. In this case, the reaction temperature usually ranges from 0°C to 30°C and the reaction time is 1~5 hours.

The quinone compound (I) as produced in the above manner can be isolated and collected by a per se known separation and purification means (e.g., chromatography, crystallization method), etc.

The hydroquinone derivative of a quinone compound (I) of this invention is represented by the general formula ;

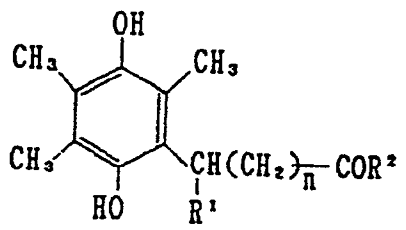

(I')

(wherein each of the symbols is as defined hereinbefore)

The compounds (I) and (I') are interchangeable with each other relative to the quinone and hydroquinone nuclei through the chemical or biochemical oxidation and reduction reactions. Since the hydroquinone compound (I') is generally susceptible to oxidation by oxygen, air, etc., the hydroquinone compound (I') is normally handled in the form of the quinone compound (I) as a stable compound. In view of the fact that the chemical and biochemical interchange between the hydroquinone compound (I') and the quinone compound (I) easily occurs, the quinone compound (I) and the hydroquinone compound (I'), when they demonstrate pharmacological activities under physiological conditions, can be regarded as possessing equivalent properties.

The quinone compound (I) can be readily converted to the hydroquinone compound (I') by conducting reduction by a per se known method with the use of a mild reducing agent, such as sodium hydrosulfite, acid sodium sulfite and sodium borohydride.

The quinone compounds (I) and (I') have, from the structural standpoint, the asymmetric center of n carbon in the side-chain of the quinon nucleus, which allows the optically active compounds to exist. Therefore, the compounds (I) and (I') of the present invention shall be meant to include any of its optically active compounds and racemic compounds.

The compounds (I) and (I') of the present invention exhibit metabolism ameliorating action for poly unsaturated fatty acids (linoleic acid, γ-linolenic acid, α-linolenic acid, arachidonic acid, dihomo-γ-linolenic acid, eicosapentaenoix acid), particularly inhibitory activity of peroxidation of fatty acids (antioxidant activity), inhibitory activity of 5-lipoxygenase metabolites (e.g., leukotrienes, 5-hydroxyeicosatetraenoic acid, 5-peroxyeicosatetraenoic acid, lipoxins, etc.), inhibitory or scavenging action of activated oxygen species (superoxide anion radical, hydroxy radical, hydrogen peroxide) which are produced in the living body. Also, they exhibit, at the same time, strong inhibitory actions of leukotriene $D_4$ or platelet aggregating factor (PAF)-induced bronchoconstrictions in guinea pigs, improvement of injury of rat kidney, or inhibitory action on induction of convulsive seizure in the experimental cerebral infarction model of spontaneously hypertensive rats (SHR). Resides, the compound (I) of this invention is very low in toxicity and shows very little undesirable side effects, and shows remarkable effects in a small dose. Consequently, the compounds (I) and (I') of the present invention can be expected to develop therapeutic and prophylactic effects in mammals (mice, rats, rabbits, monkeys, horses, man, etc.) against various diseases such as bronchial asthma, psoriasis, inflammation, immediate type allergy, arteriosclerosis, atherosclerosis, fatty liver, hepatitis, hepatocirrhosis, hyper reactive pneumonitis, immunodeficiency, ischemic heart, cerebral-, renal-circulatory disorders, diminished resistance to bacterial infections, etc. and is useful as medicines, for example, antiasthmatic agent, antiallergic agent, therapeutic agent for psoriasis, cerebral-circulatory metabolism ameliorating agent, preventive agent for coronary arteriosclerosis, immune regulating agent, protection enhancing agent against bacterial infection, prostaglandin-thromboxane metabolism ameliorating agent, and therapeutic agent for fatty liver, hepatitis, hepatocirrhosis and hyper reactive pneumonitis, etc. When $R^4$ of the compound (I) is a group containing an imidazole group, the compound and its hydroquinone derivative exhibit, in addition to the above-described actions, thromboxane synthetase inhibitory activity, and can be used as an antithrombotic agent for the purpose of prevention and treatment of, for example, thrombosis, cardiac infarction, cerebral infarction, cardiac insufficiency, arrythmia, etc.

The compound of the present invention is low in toxicity, and can be safely administered orally or parenterally, as such or as pharmaceutical compositions [e.g., tablets, capsules (inclusive of soft capsules and microcapsules), solutions, injectable solutions, suppositories] prepared by mixing them with the per se known pharmaceutically acceptable carriers, excipients, etc. Though the dosage level varies depending upon the condition of the patients to be treated, route of administration, conditions of the disease, etc., the compound, for example in the case of. oral administration to a human adult with asthma, is favorably administered at a single dose of normally about 0.1 mg/kg~20 mg/kg body weight, preferably about 0.2 mg/kg~10 mg/kg body weight, about 1~2 times/day.

The compounds (I) and (I') of the present invention have a bulky group at α-position carbon atom in the side chain of the quinone jor hydroquinone nucleus, and because of the characteristic structure, are hardly susceptible to an inactivation reaction due to in vivo metabolism. Consequently, these compounds can maintain

the effective blood concentration level of a drug and thus demonstrate improved efficacy at a lowered dosage level and long duration time as compared with known quinone compounds. The compound (I) where $R^1$ is a functional group containing an imidazole group exhibits specific dual inhibitory effect on 5-lipoxygenase and thromboxane synthetase simultaneously, and is favorable for the application as a cardiovascular drug.

The compound (II) can be produced by the method shown by the following scheme.

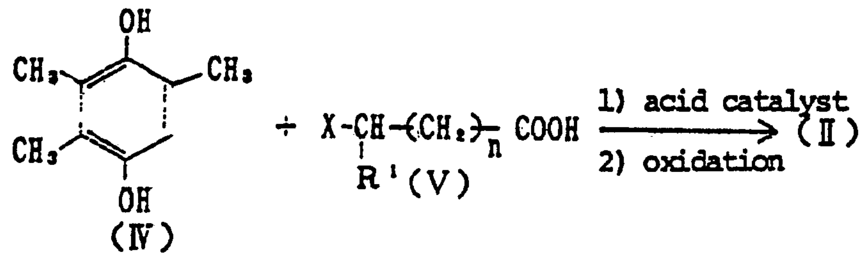

(IV) (V) (II)

(wherein $R^1$ and n are as defined hereinbefore, and X stands for a hydroxyl group, acetoxy, methoxy or halogen).

More specifically, a compound (IV) and a compound (V) are subjected to condensation in the presence of an acid catalyst, followed by subjecting the resultant condensate to oxidation.

This condensation reaction is carried out in a nonpolar solvent (e.g. methylene chloride, chloroform, benzene, toluene, isopropyl ether, 1,2-dichloroethane and 1,1,2,2-tetrachloroethane) in the presence of an acid catalyst (e.g. boron trifluoride ethyl etherate, aluminium chloride, tin chloride, p-toluenesulfonic acid, D-camphorsulfonic acid ; etc.) in the temperature range of 10°C~100°C.

The amount of the acid catalyst to be used is in the range of about 1/20 mole~3.0 mole relative to the compound (IV). This reaction is conducted preferably under oxygen-free conditions. The reaction under oxygen-free conditions yields a hydroquinone compound. This hydroquinone compound can be converted to a quinone compound (II) by oxidation to be followed. The oxidation of a hydroquinone compound is conducted by using a mild oxidizing agent such as air, oxygen, Fremy's salt, ferric chloride, ferric sulfate, hydrogen peroxide, peracid, etc. This reaction is conducted usually in the presence of a solvent. The solvent is exemplified by methanol, acetonitrile, dioxane, 1,2-dimethoxyethane and a aqueous mixture of any of these solvents. When air or oxygen is used as an oxidizing agent, the reaction is carried out while keeping the pH of the reaction solution neutral or weakly alkaline (pH 7.0~pH 9.0) by using a suitable buffer solution (e.g. phosphate buffer solution). The reaction temperature ranges from − 10°C to 30°C, and the reaction time is usually 24 hours. When ferric chloride, ferric sulfate, Fremy's salt, hydrogen peroxide or peracid (e.g. peracetic acid, m-chloro perbenzoic acid) is used as an oxidizing agent, its amount is preferably about 1~4 mole relative to 1 mole of the condensate. The reaction temperature is in the range of − 10°C~30°C, and the reaction time is usually one hour or less.

The novel quinone derivatives of the present invention possess metabolism ameliorating action for polyunsaturated fatty acids, particularly production inhibitory activity of lipid peroxides (antioxidant activity), production inhibitory activity of 5-lipoxygenase metabolites, inhibitory actions of leukotriene $D_4$ − or platelet aggregating factor-induced bronchoconstrictions and scavenging action of activated oxygen species, thus being useful as drugs such as antiasthmatic, antiallergic agent and therapeutic and ameliorating agent for cardiac −, cerebraland renal-circulatory disorders.

[Working Examples]

Example 1 (Compound No. 1)

In dichloromethane (70 ml) were dissolved 7.08 g (20 mmole) of 7-(3,5,6-trimethyl-1,4-benzoquinon-2-yl)-7-phenylheptanoic acid and 3.06 g (22 mmole) of p-nitrophenol. To the solution, while stirring under ice-cooling, was added 4.53 g (22 mmole) of dicyclohexylcarbodiimide over the period of 5 minutes. The mixture was stirred continuously for 30 minutes under ice-cooling and for one hour at room temperature. Dichloromethane was then distilled off under reduced pressure. To the residue was aded ethyl acetate (50 ml), which was left standing for 17 hours under icecooling. The insoluble matter (urea) was filtered off. The filtrate was dried (over magnesium sulfate), and the solvent was distilled off under reduced pressure. The residue was recrystallized from isopropyl ether to give 8.65 g (91%, m.p. 86~88°C) of p-nitrophenyl 7-(3,5,6-trimethyl-1,4-benzoquinon-2-yl)-7-phenylheptanoate.

To 0.71 g (1.5 mmole) of this p-nitrophenyl ester dissolved in tetrahydrofuran (7 ml) was added concentrated ammonia water (1.0 ml) at room temperature. The mixture was stirred for 4 hours. Tetrahydrofuran was distilled off under reduced pressure. To the residue were added ethyl acetate and an aqueous solution of potassium carbonate, and the extraction was conducted. The ethyl acetate layer was washed with water then with an aqueous solution of sodium chloride, and dried (magnesium sulfate). Ethyl acetate was distilled off under reduced pressure ; and the residue was recrystallized from isopropyl ether to give 0.46 g of 7-(3,5,6-trimethyl-1,4-benzoquinon-2-yl)-7-phenylheptanamide. By following the procedure of this Example, the Compound Nos. 2 to 17 set forth in Table 1 were prepared.

Example 2 (Compound No. 18)

In dichloromethane (8 ml) were dissolved 0.50 g (1.05 mmole) of p-nitrophenyl 7-(3,5,6-trimethyl-1,4-benzoquinon2-yl)-7-phenyl heptanoate and 0.20 g (1.05 mmole) of 4-(2-phenylethyl)piperazine, and the solution was stirred for 18 hours at room temperature. Dichloromethane was distilled off under reduced pressure. The residue was subjected to extraction by the addition of ethyl acetate and an aqueous solution of potassium carbonate. The ethyl acetate layer was washed with water then with an aqueous solution of sodium chloride and dried (magnesium sulfate), the resultant was concentrated under reduced pressure. The concentrate was purified by means of silica gel column chromatography [eluting with ethyl acetate and chloroform/methanol (10: 1), successively] to give 0.51 g of 7-(3,5,6-trimethyl-1,4-benzoquinon-2-yl)-7-phenylheptanoic acid 4-(2-phenylethyl)-piperazinamide.

Example 3 (Compound No. 19)

To a mixture of 0.95 g (2.0 mmole) of p-nitrophenyl 7-(3,5,6-trimetyl-1,4-benzoquinon–2-yl)-7-phenylheptanoate and 0.71 g (2.1 mmole) of glycine benzyl ester·p-toluene sulfonate were added dichloromethane (10 ml) and 0.29 ml (2.1 mmole) of triethylamine to make a solution, which was stirred at room temperature for 2.5 days. Dichloromethane was distilled off under reduced pressure. The residue was subjected to extraction by the addition of ethyl acetate and an aqueous solution of potassium carbonate. The ethyl acetate layer was washed with water and an aqueous solution of sodium chloride, followed by drying (magnesium sulfate). The resultant was concentrated under reduced pressure and subjected to purification by means of silica-gel column chromatography [eluting with isopropyl ether, ethyl acetate/ isopropylether(1 : 1), successively] to give 0.78 g of 7-(3,5,6-trimethyl-1,4-benzoquinon-2-yl)-7-phenylheptanoyl glycine benzyl ester.

Example 4 (Compound No. 20)

To benzyl ester of 7-(3,5,6-trimethyl-1,4-benzoquinon-2-yl)-7-phenylheptanoyl glycine (0.78 g) dissolved in ethanol (8 ml) was added 5% palladium-carbon (0.08 g), which was subjected to catalytic reduction at room temperature for 3 hours. The catalyst was filtered off. To the filtrate was added a solution of ferric chloride (0.51 g) in water (5 ml), and the mixture was stirred at room temperarure for 20 minutes. Ethanol was distilled off under reduced pressure. The residue was subjected to extraction by the addition of ethyl acetate and water. The ethyl acetate layer was washed with an aqueous solution of sodium chloride and dried (magnesium sulfate), followed by concentration under reduced pressure. Precipitating crystals were collected by filtration and washed with isopropyl ether to give 0.63 g of 7-(3,5,6-trimethyl-1,4-benzoquinon-2-yl)-7-phenylheptanoylglycine.

By following the procedure of the above Example 3 and 4, the Compound Nos 21~27, 29 and 30 were prepared.

Example 5 (Compound No. 28)

To a mixture of 0.95 g (2.0 mmole) of p-nitrophenyl-ester of 7-(3,5,6-trimethyl-1,4-benzoquinon-2-yl)-7-phenylheptanoic acid and 0.24 g (2.1 mmole) of L-proline were added dimethyl- formamide (10 ml) and 0.48 g (4.2 mmole) of N-ethyl-morpholine to make a solution, which was stirred at room temperature for 15 hours, followed by addition of 1N hydrochloric acid (5 ml). The mixture was subjected to extraction by the addition of ethyl acetate and an aqueous solution of sodium chloride. The ethyl acetate layer was washed with an aqueous solution of sodium chloride and dried (magnesium sulfate). From the resultant was distilled off ethyl acetate under reduced pressure, and the residue was subjected to a column chromatography using YMC-GEL ODS (60/200 mesh, manufactured by Yamanura Kagaku Kenkyūjo), followed by purification (eluting with 90% methanolic water) to obtain fractions containing the end product. Methanol was distilled off, and the residue was subjected to extraction with ethyl acetate. The ethyl acetate layer was washed with an aqueous solution

of sodium chloride and dried (magnesium sulfate). Ethyl acetate was distilled off under reduced pressure to give 0.64 g of 7-(3,5,6-trimethyl-1,4-benzoquinon-2-yl)-7-phenylheptanoyl-L-proline.

Example 6 (Compound No. 31)

A solution of 0.71 g of (7R)-(+)-7-(3,5,6-trimethyl1,4-benzoquinon-2-yl)-7-phenylheptanoic acid in anhydrous tetrahydrofuran (10 ml) was cooled to – 10°C, to which was added, under argon atmosphere, a solution of 0.24 g of ethyl chlorocarbonate in tetrahydrofuran (2 ml), followed by addition of a solution of 0.31 ml of triethylamine in tetrahydrofuran (2 ml) dropwise over a period of 5 minutes. The mixture was stirred for further 15 minutes at – 10°C, to which was then added 0.36 g of a solution of p-bromoaniline in tetrahydrofuran (4 ml) dropwise over a period of 5 minutes. The mixture was stirred for a further 1 hour at – 100°C to 0°C, then tetrahydrofuran was distilled off under reduced pressure. The residue was subjected to extraction by the addition of ethyl acetate and water. The ethyl acetate layer was washed with 1 N hydrochloric acid, an aqueous solution of potassium carbonate and an aqueous saline solution, successively, followed by drying (magnesium sulfate). The resultant was concentrated under reduced pressure, and precipitating crystals were collected by filtration, followed by washing with isopropylether to obtain 0.77 g of p-bromoanilide (7R)-(4)-7-(3,5,6-trimethyl-1,4-benzoquninon-2-yl)-7-phenylheptanoate [$[\alpha]_D^{22}$ = + 18.5° (c = 1, chloroform)].

Table 1

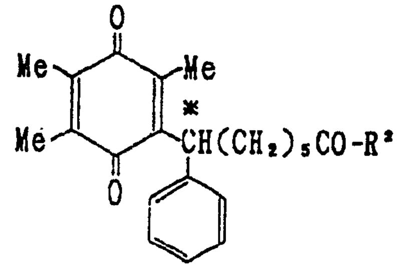

| Compound No. | Prepared by the procedure by Example | $R^2$ | Molecular formula Molecular weight | Melting point (°C) Physical properties |
|---|---|---|---|---|
| 1 | 1 | $-NH_2$ | $C_{22}H_{27}NO_3$ 353.46 | 105-106 |
| 2 | 1 | $-NHPr^i$ | $C_{25}H_{33}NO_3$ 395.54 | 99-101 |
| 3 | 1 | 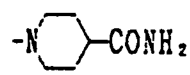 | $C_{28}H_{36}N_2O_4$ 464.61 | oil |
| 4 | 1 | 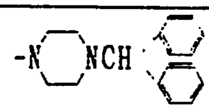 | $C_{39}H_{44}N_2O_3$ 588.79 | oil |
| 5 | 1 | 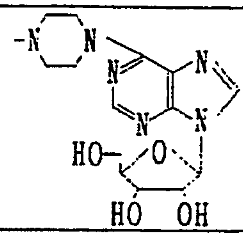 | $C_{36}H_{44}N_6O_7$ 672.78 | amorphous |
| 6 | 1 | 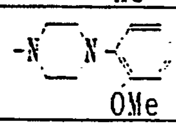 | $C_{33}H_{40}N_2O_4$ 528.69 | oil |
| 7 | 1 | 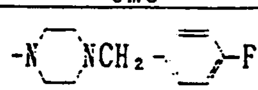 | $C_{33}H_{39}FN_2O_3$ 530.68 | oil |
| 8 | 1 | 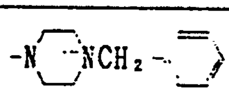 | $C_{33}H_{40}N_2O_3$ 512.69 | oil |

Table 1 (continued)

| Compound No. | Prepared by the procedure by Example | $R^2$ | Molecular formula Molecular weight | Melting point (°C) Physical properties |
|---|---|---|---|---|
| 9 | 1 | 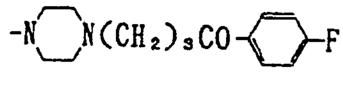 | $C_{36}H_{43}FN_2O_4$ 586.75 | oil |
| 10 | 1 | 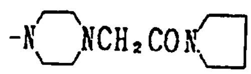 | $C_{32}H_{43}N_3O_4$ 533.71 | oil |
| 11 | 1 | 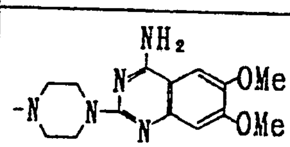 | $C_{38}H_{43}N_5O_5$ 625.77 | 207-209 |
| 12 | 1 | 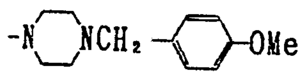 | $C_{34}H_{42}N_2O_4$ 542.72 | oil |
| 13 | 1 | 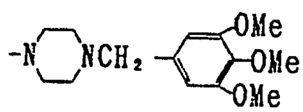 | $C_{36}H_{46}N_2O_6$ 602.77 | oil |
| 14 | 1 | 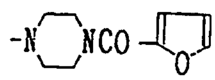 | $C_{31}H_{36}N_2O_5$ 516.64 | oil |
| 15 | 1 | *1 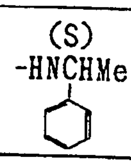 | $C_{30}H_{35}NO_3$ 457.61 | oil |
| 16 | 1 | *2 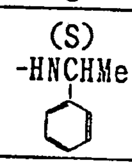 | $C_{30}H_{35}NO_3$ 457.61 | oil |
| 17 | 1 | 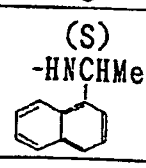 | $C_{34}H_{37}NO_3$ 507.67 | oil |

Table 1 (continued)

| Compound No. | Prepared by the procedure by Example | $R^2$ | Molecular formula Molecular weight | Melting point (°C) Physical properties |
|---|---|---|---|---|
| 18 | 2 | 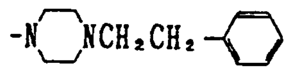 | $C_{34}H_{42}N_2O_3$ 526.72 | oil |
| 19 | 3 | -GlyOCH$_2$ 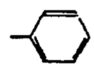 | $C_{31}H_{35}NO_5$ 501.62 | oil |
| 20 | 4 | -GlyOH | $C_{24}H_{29}NO_5$ 411.50 | 162-164 |
| 21 | 3 | -Arg(NO$_2$)OBzl | $C_{35}H_{43}N_5O_7$ 645.76 | oil |
| 22 | 4 | -ArgOH · HCl | $C_{28}H_{38}N_4O_5 \cdot HCl$ 547.10 | amorphous |
| 23 | 3, 4 | -HisOH | $C_{28}H_{33}N_3O_5$ 491.59 | amorphous |
| 24 | 3 | -Asp(OBzl)OBzl | $C_{40}H_{43}NO_7$ 649.78 | oil |
| 25 | 4 | -AspOH | $C_{26}H_{31}NO_7$ 469.53 | amorphous |
| 26 | 3 | -Glu(OBzl)OBzl | $C_{41}H_{45}NO_7$ 663.81 | oil |

Table 1 (continued)

| Compound No. | Prepared by the procedure by Example | $R^2$ | Molecular formula Molecular weight | Melting point (°C) Physical properties |
|---|---|---|---|---|
| 27 | 4 | -GluOH | $C_{27}H_{33}NO_7$ 483.56 | amorphous |
| 28 | 5 | -ProOH | $C_{27}H_{33}NO_5$ 451.56 | oil |
| 29 | 3 | -PheOBzl | $C_{38}H_{41}NO_5$ 591.75 | oil |
| 30 | 4 | -PheOH | $C_{31}H_{35}NO_5$ 501.62 | amorphous |
| 31 | 6 | *1 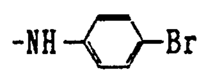 | $C_{28}H_{30}BrNO_3$ 508.46 | 163-164 |

Table 1 (continued)

| Compound No. | Nuclear magnetic resonance spectrum, δ value (ppm), TMS as internal standard |
|---|---|
| 1 | 1.1-1.8(6H),1.9-2.4(4H),1.97(6H),2.04(3H), 4.29(1H),5.95(2H),7.24(5H) |
| 2 | 1.11(6H),1.1-1.8(6H),1.9-2.3(4H),1.96(6H), 2.03(3H),4.05(1H),4.29(1H),5.50(1H),7.23(5H) |
| 3 | 1.1-1.8(6H),1.7-2.4(9H),1.97(6H),2.03(3H), 2.65(1H),3.02(1H),3.86(1H),4.28(1H),4.54(1H), 5.78(2H),7.24(5H) |
| 4 | 1.1-1.8(6H),1.9-2.3(4H),1.95(6H),2.02(3H), 2.34(4H),3.42(2H),3.59(2H),4.22(1H),4.28(1H), 7.1-7.5(15H) |
| 5 | 1.1-1.8(6H),1.9-2.4(4H), 1.95(6H),2.03(3H),3.58 (4H),3.5-4.5(10H),4.8-5.2(1H),4.94(1H),5.81(1H), 7.23(5H),7.86(1H),8.14(1H) |
| 6 | 1.1-1.8(6H), 1.9- 2.4(4H), 1.97(6H), 2.05(3H), 3.00(4H),3.61(2H),3.86(3H),3.77(2H),4.30(1H), 6.92(4H),7.26(5H) |

Table 1 (continued)

| Compound No. | Nuclear magnetic resonance spectrum, δ value (ppm), TMS as internal standard |
|---|---|
| 7 | 1.1-1.8(6H), 1.9-2.4(4H), 1.97(6H), 2.04(3H), 2.37(4H), 3.41(2H), 3.45(2H), 3.58(2H), 4.29(1H), 6.98(4H),7.1-7.4(2H),7.25(5H) |
| 8 | 1.1-1.8(6H),1.9-2.4(4H),1.97(6H),2.03(3H), 2.38(4H),3.41(2H),3.48(2H),3.59(2H),4.29(1H), 7.24(5H), 7.30(5H) |
| 9 | 1.1-1.8(6H),1.8-2.5(12H),1.98(6H),2.04(3H), 2.97(2H),3.37(2H),3.51(2H),4.30(1H),7.12(2H), 7.26(5H),7.99(2H) |
| 10 | 1.1-1.8(6H),1.7-2.4(8H),1.97(6H),2.04(3H), 2.54(4H),3.13(2H),3.3-3.7(8H),4.29(1H),7.25(5H). |
| 11 | 1.1-1.8(6H),1.9-2.4(4H),1.95(6H),2.02(3H), 3.4-4.0(8H),3.87(3H),3.94(3H),4.30(1H), 5.40(2H),6.90(2H),7.24(5H) |
| 12 | 1.1-1.8(6H),1.9-2.4(4H), 1.96(6H), 2.03(3H),2.36(4H),3.39(2H),3.42(2H),3.59(2H),3.77(3H), 4.30(1H),6.83(2H),7.21(5H),7.24(5H) |
| 13 | 1.1-1.8(6H), 1.9-2.4(4H), 1.96(6H), 2.03(3H), 2.39(4H),3.3-3.7(4H),3.41(2H),3.82(3H),3.85(6H), 4.29(1H),6.55(2H),7.24(5H) |

Table 1 (continued)

| Compound No. | Nuclear magnetic resonance spectrum, δ value (ppm), TMS as internal standard |
|---|---|
| 14 | 1.1-1.8(6H), 1.9-2.4(4H), 1.96(6H), 2.03(3H), 3.4-3.9(8H), 4.31(1H), 6.48(1H), 7.04(5H),7.24 (5H), 7.48(1H) |
| 15 | 1.1-1.8(6H),1.44(3H),1.97-2.3(4H),1.96(6H),2.02 (3H),4.26(1H),5.09(1H),5.92(1H),7.23(5H),7.27(5H) |
| 16 | 1.1-1.8(6H),1.45(3H),1.9-2.3(4H),1.96(6H) 2.02(3H),4.27(1H),5.10(1H),5.91(1H),7.22(5H) 7.27(5H) |
| 17 | 1.1-1.8(6H),1.59(3H),1.9-2.3(4H),1.93(6H),2.00 (3H), 4.23(1H),5.95(1H),6.00(1H),7.23(5H),7.3-7.5(4H), 7.78(2H),8.07(1H); |
| 18 | 1.1-1.8(6H),1.9-2.9(12H),1.97(6H),2.04(3H), 3.46(2H),3.63(2H),4.30(1H),7.22(5H), 7.24(5H) |
| 19 | 1.1-1.8(6H),1.9-2.3(4H),1.98(6H), 2.05(3H), 4.07 (2H),4.30(1H),5.19(2H),6.03(1H),7.28(5H),7.39(5H) |
| 20 | 1.1-1.8(6H), 1.9-2.3(4H), 1.97(6H), 2.03(3H), 4.02(2H),4.28(1H),6.39(1H),7.23(5H),7.60(1H), |

Table 1 (continued)

| Compound No. | Nuclear magnetic resonance spectrum, δ value (ppm), TMS as internal standard |
|---|---|
| 21 | 1.1-1.9(10H), 1.9-2.5(6H), 1.95(6H), 2.02(3H), 3.30(2H), 4.27(1H), 4.64(1H), 5.15(2H), 6.55(1H) 7.24(5H), 7.32(5H), 7.68(2H), 8.32(1H) |
| 22 | 1.0-1.8(10H), 1.9-2.4(6H), 1.93(3H), 1.99(3H), 3.27 (2H), 4.24(1H), 4.48(1H), 6.8-7.2(1H), 7.29(5H), 7.63 (1H), 8.13(3H) |
| 23 | 1.0-1.7(6H), 1.9-2.3(4H), 1.93(6H), 1.99(3H), 3.09 (2H), 4.23(1H), 4.48(1H), 6.90(1H), 7.0-7.5(6H), 8.26(1H), 11.24(2H) |
| 24 | 1.1-1.8(6H), 1.9-2.3(4H), 1.96(6H), 2.04(3H), 2.95(2H), 4.28(1H), 4.89(1H), 5.04(2H), 5.12(2H), 6.48(1H), 7.25(5H), 7.30(10H). |
| 25 | 1.1-1.8(6H), 1.9-2.3(4H), 1.96(6H), 2.03(3H), 2.92(2H), 4.27(1H), 4.83(1H), 6.62(2H), 6.97(1H), 7.24(5H) |
| 26 | 1.1-1.8(6H), 1.8-2.5(8H), 1.96(6H), 2.04(3H), 4.27 (1H), 4.64(1H), 5.07(2H), 5.14(2H), 6.18(1H), 7.25(5H) 7.33(10H) |
| 27 | 1.1-1.8(6H), 1.9-2.6(8H), 1.95(6H), 2.03(3H), 4.27(1H), 4.59(1H), 6.86(1H), 7.21(5H), 8.23(2H). |

Table 1 (continued)

| Compound No. | Nuclear magnetic resonance spectrum, δ value (ppm), TMS as internal standard |
|---|---|
| 28 | 1.1-1.8(6H),1.9-2.4(8H),1.97(6H),2.03(3H),3.50(2H),4.30(1H),4.54(1H),7.1-7.5(1H),7.23(5H) |
| 29 | 1.1-1.8(6H),1.9-2.3(4H),1.96(6H),2.02(3H),3.09(2H), 4.27(1H),4.92(1H),5.13(2H),5.84(1H),6.9-7.4(5H), 7.24(5H),7.32(5H) |
| 30 | 1.1-1.8(6H),1.9-2.3(4H),1.96(6H),2.03(3H), 3.14(2H),4.26(1H),4.83(1H),6.15(1H),7.17(5H), 7.23(5H),7.39(1H) |
| 31 | 1.1-1.9(6H),1.9-2.4(4H), 1.96(6H), 2.03(3H), 4.29(1H),7.23(5H),7.38(4H), |

In the above Table, *1 and *2 in the column of $R^2$ show the optical isomer at the position marked with * in the structural formula shown at the head of the table and the optical rotation thereof. The compound bearing with *1 means an optical isomer (R)-(+) and that bearing with *2 means (S)-(–). Gly, Arg, His, Asp, Pro, Phe, Bzl and Glu stand for glycyl, arginyl, histidyl, aspartyl, prolyl, phenylalanyl, benzyl and glutamyl, respectively.

Example 7

Pharmaceutical Composition

A) Capsule

| | |
|---|---|
| (1) Compound No. 1 | 50 mg |
| (2) Cellulose fine powder | 30 mg |
| (3) Lactose | 37 mg |
| (4) Magnesium stearate | 3 mg |
| Total | 120 mg |

All the materials were blended and filled into a gelatin capsule.

B) Soft Capsule

| | |
|---|---|
| (1) Compound No. 30 | 50 mg |
| (2) Corn oil | 100 mg |
| Total | 150 mg |

C) Tablet

| | |
|---|---|
| (1) Compound No. 1 | 50 mg |
| (2) Lactose | 34 mg |
| (3) Corn starch | 10.6 mg |
| (4) Corn starch (gelatinized) | 5 mg |
| (5) Magnesium stearate | 0.4 mg |
| (6) Calcium carboxymethyl cellulose | 20 mg |
| Total | 120 mg |

All the materials were blended and compressed by a tabletting machine to prepare a tablet in accordance with a conventional manner.

Experimental Example 1

Platelet activating factor (PAF)-induced bronchoconstriction in guinea pigs

Bronchoconstriction in guinea pigs induced by PAF (1 µg/kg, intravenous injection) was measured by the method of Konzett-Rossler. The following procedure was the same as that in leukotriene $D_4$($LTD_4$)-induced bronchoconstriction. Test drugs were orally administered as a 5% gum arabic solution one hour before administration of PAF. Table 2 Inhibitory action against bronchoconstriction in guinea pigs

| Compound No. | Dose mg/kg | Number of animals | % inhibition |
|---|---|---|---|
| | 0.313 | 8 | 49.7* |
| 1 | 1.25 | 8 | 80.3** |
| | 5 | 8 | 100.0** |
| 18 | 5 | 8 | 59.9* |
| 30 | 5 | 8 | 72.1* |

*P<0.05, **P<0.01 : reliability against control group

Reference Example 1

To monoethyl pimelate (120 g, 0.638 mole) was added thionyl chloride (92 ml). The mixture was heated at 50°C for 2 hours. After cooling, excess thionyl chloride was removed under reduced pressure. The residue was subjected to distillation under reduced pressure to obtain 129.4 g (98%) of ethyl pimelic acid chloride, b.p.(0.3 mm Hg) 90-91°C. This chloride (41.4 g, 0.20 mole) was dissolved in benzene (71.0 ml), to which was gradually added aluminium chloride (53.2 g, 0.40 mole). The reaction solution was stirred at room temperature for 20 minutes, then at 90°C for 2 hours, the solution was then poured into ice-water (500 ml). To this solution were added concentrated hydrochloric acid (40 ml) and ethyl acetate (500 ml) and the mixture was stirred. The organic layer was separated, washed with water, dried and concentrated. The concentrate was dissolved in ethanol (150 ml), to which was added concentrated sulfuric acid. The mixture was stirred at 90°C for 2 hours, cooled by aeration, neutralized with sodium hydrogencarbonate, then the solvent was distilled off. The residue was subjected to extraction with ethyl acetate. The extract was washed with water, dried and concentrated to give ethyl ketocarboxylate (50.3 g). This product was dissolved in methanol (300 ml), to which was added sodium boronhydride (4.5 g) little by little. The reaction solution was stirred at room temperature for one hour. Excess amount of the reagent was decomposed with acetone, followed by addition of water (400 ml) ; which was subjected to extraction with ethyl acetate. The organic layer was washed with water, dried and concentrated under reduced pressure. The residue was dissolved in a mixture of tetrahydrofuran (150 ml) and water (150 ml), to which was added sodium hydroxide (20 g). The mixture was stirred at 70°C for 3 hours. The reaction solution was concentrated under reduced pressure, followed by addition of 2N hydrochloric acid to render the pH to 3.0. The resultant was subjected to extraction with ethyl acetate

The organic layer was washed with water, dried and concentrated under reduced pressure to obtain 7-hydroxy-7-phenylheptanoic acid as an oily product (42.2 g, 95%). NMR (in $CDCl_3$) : δ 1.1-1.8(8H), 2.29(2H), 4.63(1H), 7.30(5H)

Reference Example 2

To toluene (570 ml) were added 2,3,5-trimethyl hydroquinone (28.9 g, 0.19 mole) and 7-hydroxy-7-phenylheptanoic acid (42.2 g, 0.19 mole). To the mixture was added dropwise while stirring boron trifluoride ethyl etherate (8.1 g) at 60°C over a period of 20 minutes. The reaction solution was stirred for 2.5 hours under the same conditions, followed by distilling off the solvent under reduced pressure. The residue was dissolved in tetrahydrofuran (300 ml), to which were added ferric chloride (102.6 g) and water (300 ml) to oxidize into a quinone compound. The product was extracted twice with ethyl acetate. The organic layer was washed with water, dried and concentrated under reduced pressure. The crude product thus obtained was subjected to silica gel column chromatography, eluting with isopropyl ether. The quinone compound was recrystallized from ethanol (250 ml) to give 7-(3,5,6-trimethyl-1,4-benzoquinon-2-yl)-7-phenylheptanoic acid (49.0 g, 73%), m.p.128~129°C.

Reference Example 3

In ethyl acetate (142 ml) was dissolved (±)-7-(3,5,6trimethyl-1,4-benzoquinon-2-yl)-7-phenylheptanoic acid (7.08 g, 20 mmole). To the solution was added dropwise at room temperature 2.57 ml (20 mmole) of L-(-) -phenylethylamine over a period of 5 minutes, and the mixture was stirred for one hour. Precipitating crystals were collected by filtration and suspended in ethyl acetate (100 ml). To the suspension was added 1N hydrochloric acid (30 ml), which was stirred for 15 minutes. The ethyl acetate layer was taken out, was washed with an aqueous saline solution and dried (magnesium sulfate). The solvent was distilled off to give a compound consisting of predominantly (+) compound.

The compound thus obtained was subjected to the abovementioned procedure four times repeatedly to give a (+)optically active compound (1.36 g). $[\alpha]_D^{22}$ = +23.6° (c = 1, chloroform). This compound was recrystallized from ethanol (6.8 ml), and the precipitates were collected by filtration. The solvent was distilled off, and the residue was crystallized from isopropyl ether to give a (+)- optically active compound, (+)-7-(3,5,6-trimethyl-1,4-benzoquinon-2-yl)-7phenylheptanoic acid (1.10 g), m.p.79~82°C. The yield was 16%. $[\alpha]_D^{22}$ = +24.4° (c = 1, chloroform)

On the other hand, using D-(+)-α-phenylethylamine, a similar procedure to the above was followed to thereby obtain a (-)-optically active compound, (-)-7-(3,5,6-trimethyl-1,4-benzoquinon-2-yl)-7-phenylheptanoic acid (1,20 g) from 7.08 g of the compound N°. 50. The yield was 17%. $[\alpha]_D^{22}$ = −24.4° (c = 1, chloroform) m.p.79~82°C

## Claims

### Claims for the Contracting States BE CH DE FR GB IT LI LU NL SE GR

1. A compound of the general formula :

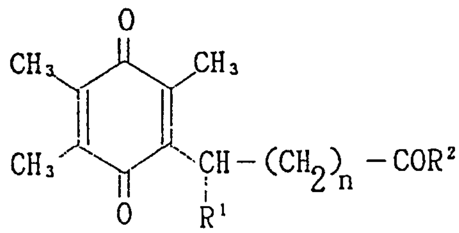

(I)

wherein
$R^1$ is phenyl unsubstituted or a phenyl substituted at an optional position on the ring with
hydroxyl,
halogen,
alkyl having 1 to 3 carbon atoms or
alkoxy having 1 to 3 carbon atoms,
$R_2$ is amino unsubstituted, an amino substituted with
$(C_{1-4})$alkyl
$(C_{6-10})$aryl unsubstituted,
$(C_{6-10})$aryl substituted with
hydroxyl,
amino,
nitro,
halogen,
methyl or
methoxy,
benzyl,
α-phenethyl,
β-phenethyl,
1-(α-naphthyl)ethyl,
amino acid residue resulting from eliminating one hydrogen from the amino the group consisting of
glycine,
arginine,
histidine,
aspartic acid,
proline,
phenylalanine,
methionine,
alanine,
leucine and
glutamic acid
the amino acid residue being unesterified or esterified at the carboxyl group with
benzyl alcohol or
the amino acid residue being unprotected or protected, when the amino acid residue has an amino group, with
nitro
cyclic amino selected from a group consisting of
morpholino,
piperidino,
piperazino or
pyrrolidino,
the cyclic amino being unsubstituted or substituted with
methyl,

ethyl,
propyl
pyrrolidinocarbonylmethyl,
4-fluorophenylcarbonylpropyl,
phenyl,
naphthyl,
2-methoxyphenyl,
4-methylphenyl,
4-bromophenyl,
benzyl,
2-phenylethyl,
4-fluorophenylmethyl,
4-methoxyphenylmethyl,
3,4,5-trimethoxyphenylmethyl,
diphenylmethyl,
aminocarbonyl,
6-(9-β-D-ribofuranoside)adenyl or
4-amino-6,7-dimethoxyquinazolinyl,
2-furylcarbonyl

and n is an integer of 3 to 10, or a hydroquinone derivative thereof.

2. A compound as claimed in claim 1, wherein n is 4, 5 or 6.

3. A compound as claimed in claim 1, wherein $R^2$ is amino, carboxymethylamino or 4-phenethylpiperidino.

4. A compound as claimed in claim 1, wherein $R^1$ is phenyl or a phenyl having an halogen, methyl or methoxy at an optional position on the phenyl ring.

5. A compound as claimed in claim 1, wherein $R^1$ is phenyl or a phenyl having an halogen at the meta or para-position on the phenyl ring.

6. A compound as claimed in claim 1, in which $R^2$ is aralkyl selected from benzyl, α-phenethyl, β-phenethyl and 1-(α-naphthyl)ethyl.

7. A compound as claimed in claim 1, in which $R^2$ is said amino acid residue as defined in claim 1.

8. A compound as claimed in claim 1, in which $R^2$ is a cyclic amino group selected from piperazino and pyrrolidino, said cyclic amino group being unsubstituted or substituted as specified in claim 1.

9. A compound as claimed in claim 1, wherein the compound is 7-(3,5,6-trimethyl-1-,4-benzoquinone-2-yl)7-phenylheptanamide.

10. A compound as claimed in claim 1, wherein the compound is 7-(3,5,6-trimethyl-1-,4-benzoquinone-2-yl)7-phenylheptanoylglycine.

11. A compound as claimed in claim 1, wherein the compound is 1-[7-(3,5,6-trimethyl-1-,4-benzoquinone-2-yl)-7-phenylheptanoyl] 4-(2-phenylethyl)piperidine.

12. A method of preparing a quinone derivative of the general formula (I) as defined in claim 1, or a hydroquinone derivative thereof, which comprises :

(i) reacting an activator of carboxylic acid to react with a compound of the general formula (II) :

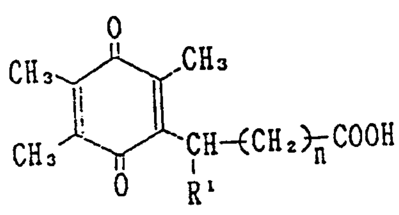

(II)

wherein $R^1$ and n are as defined in claim 1 to form a reactive derivative at the carboxyl group ; and, thereafter,

(ii) reacting the resulting compound with a compound of the general formula :

$R^2$-H

wherein $R^2$ is as defined $R^2$-H in claim 1, and, if desired, converting said quinone compound (I) to a hydroquinone derivative thereof.

13. A pharmaceutical composition, which comprises as an effective component a quinone compound (I)

as defined in any of claims 1 to 12, or a hydroquinone derivative thereof, of the general formula (I')

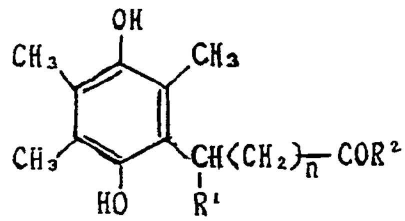

(I ')

wherein each of the symbols is as defined hereinbefore, together with a pharmaceutically acceptable carrier or diluent therefor.

14. A pharmaceutical composition as claimed in claim 13, which is a therapeutic agent for the treatment of asthma or arteriosclerosis or an antiallergic agent.

15. The use of a compound (I) as defined in any of claims 1 to 12, or a hydroquinone derivative thereof, for the manufacture of a therapeutic agent for the treatment of asthma or arteriosclerosis, or of an antiallergic agent.

**Claims for contracting stages AT, ES**

1. The use for the manufacture of a therapeutic agent for the treatment of asthma or arteriosclerosis, or an antiallergic agent, of a quinone compound of the general formula (I) :

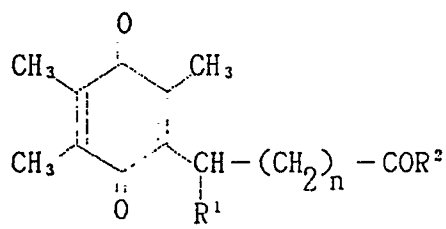

(I)

wherein
$R^1$ is phenyl unsubstituted or a phenyl substituted at an optional position on the ring with
hydroxyl,
halogen,
alkyl having 1 to 3 carbon atoms or
alkoxy having 1 to 3 carbon atoms,
$R_2$ is amino unsubstituted, an amino substituted with
$(C_{1-4})$alkyl
$(C_{6-10})$aryl unsubstituted,
$(C_{6-10})$aryl substituted with
hydroxyl,
amino,
nitro,
halogen,
methyl or
methoxy,
benzyl,
α-phenethyl,
β-phenethyl,
1-(α-naphthyl)ethyl,
amino acid residue resulting from
eliminating one hydrogen from the amino group of an amino acid selected from the group consisting of
glycine,
arginine,
histidine,
aspartic acid,
proline,

phenylalanine,
methionine,
alanine,
leucine and
glutamic acid
the amino acid residue being unesterified or esterified at the carboxyl group with
benzyl alcohol or
the amino acid residue being unprotected or protected, when the amino acid residue has an amino group, with nitro
cyclic amino selected from a group consisting of
morpholino,
piperidino,
piperazino or
pyrrolidino,
the cyclic amino being unsubstituted or substituted with
methyl,
ethyl,
propyl,
pyrrolidinocarbonylmethyl,
4-fluorophenylcarbonylpropyl,
phenyl,
naphthyl,
2-methoxyphenyl,
4-methylphenyl,
4-bromophenyl,
benzyl,
2-phenylethyl,
4-fluorophenylmethyl,
4-methoxyphenylmethyl,
3,4,5-trimethoxyphenylmethyl,
diphenylmethyl,
aminocarbonyl,
6-(9-β-D-ribofuranoside)adenyl or
4-amino-6,7-dimethoxyquinazolinyl,
2-furylcarbonyl
and n is an integer of 3 to 10, or a hydroquinone derivative thereof.

2. A method of preparing a quinone derivative of the general formula (I) as defined in claim 1, or a hydroquinone derivative thereof, which comprises :

(i) reacting an activator of carboxylic acid to react with a compound of the general formula (II) :

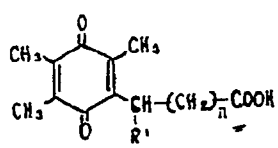

(II)

wherein $R^1$ and n are as defined in claim 1 to form a reactive derivative at the carboxyl group ; and, thereafter ;

(ii) reacting the resulting compound with a compound of the general formula :

$$R^2\text{-}H$$

wherein $R^2$ is as defined in claim 1, and, if desired, converting said quinone compound (I) to a hydroquinone derivative thereof.

3. A method as claimed in claim 2, wherein n is 4, 5 or 6.

4. A method as claimed in claim 2, wherein $R^2$ is amino, carboxyethylamino or 4-phenethylpiperidino.

5. A method as claimed in claim 2, wherein $R^1$ is phenyl or a phenyl having an halogen, methyl or methoxy at an optional position on the phenyl ring.

6. A method as claimed in claim 2, wherein $R^1$ is phenyl or a phenyl having an halogen at the meta or para-position on the phenyl ring.

7. A method as claimed in claim 2, in which $R^2$ is aralkyl selected from benzyl, α-phenethyl, β-phenethyl and 1-(α-naphthyl)ethyl.

8. A method as claimed in claim 2, in which $R^2$ is said amino acid residue as defined in claim 1.

9. A method as claimed in claim 2, in which $R^2$ is a cyclic amino group selected from piperazino and pyrrolidino, said cyclic amino group being unsubstituted or substituted as specified in claim 1.

10. A method as claimed in claim 2, wherein the compound is 7-(3,5,6-trimethyl-1-,4-benzoquinone-2-yl)-7-phenylheptanamide.

11. A method as claimed in claim 2, wherein the compound is 7-(3,5,6-trimethyl-1-,4-benzoquinone-2-yl)7-phenylheptanoylglycine.

12. A method as claimed in claim 2, wherein the compound is 1-[7-(3,5,6-trimethyl-1-,4-benzoquinone-2-yl)-7-phenylheptanoyl] 4-(2-phenylethyl)piperidine.

13. A pharmaceutical composition, which comprises as an effective component a quinone compound (I) as defined in any of claims 1 to 12, or a hydroquinone derivative thereof, of the general formula (I')

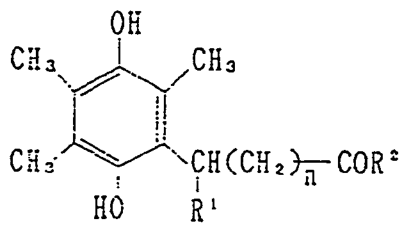

(I')

wherein each of the symbols is as defined hereinbefore, together with a pharmaceutically acceptable carrier or diluent therefor.

14. A pharmaceutical composition as claimed in claim 13, which is a therapeutic agent for the treatment of asthma or arteriosclerosis or an antiallergic agent.

## Ansprüche

### Patentansprüche für die benannten Vertragsstaaten BE CH DE FR GB IT LI LU NL SE GR

1. Verbindung der allgemeinen Formel

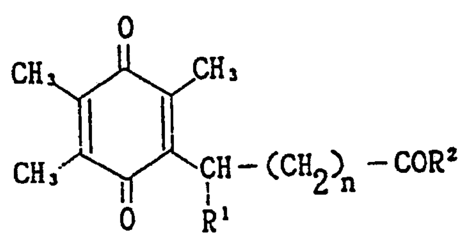

(I)

in der

$R^1$ unsubstituiertes Phenyl oder Phenyl ist, das in einer wahlfreien Position am Ring mit
- Hydroxyl,
- Halogen,
- Alkyl mit 1 bis 3 Kohlenstoff-Atomen oder
- Alkoxy mit 1 bis 3 Kohlenstoff-Atomen substituiert ist,

$R^2$ unsubstituiertes Amino, Amino, das substituiert ist mit
- $(C_{1-4})$-Alkyl,
- unsubstituiertem $(C_{6-10})$-Aryl,
- $(C_{6-10})$-Aryl, das substituiert ist mit
  - Hydroxyl,
  - Amino,

Nitro,
Halogen,
Methyl oder
Methoxy,
Benzyl,
α-Phenethyl,
β-Phenethyl,
1-(α-Naphthylethyl),
einem Aminosäure-Rest, der von der Eliminierung eines Wasserstoffs der Amino-Gruppe einer Aminosäure aus der aus
Glycin,
Arginin,
Histidin,
Asparaginsäure,
Prolin,
Phenylalanin,
Methionin,
Alanin,
Leucin und
Glutaminsäure
bestehenden Gruppe herrührt, wobei der Aminosäure-Rest unverestert oder an der Carboxy-Gruppe mit Benzylalkohol verestert ist oder
der Aminosäure-Rest ungeschützt oder, wenn der Aminosäure-Rest eine Amino-Gruppe besitzt, durch Nitro geschützt ist,
cyclischem Amino, das aus der aus
Morpholino,
Piperidino,
Piperazino oder
Pyrrolidino
bestehenden Gruppe ausgewählt ist, wobei das cyclische Amino unsubstituiert oder substituiert ist mit
Methyl,
Ethyl,
Propyl,
Pyrrolidinocarbonylmethyl,
4-Fluorophenylcarbonylpropyl,
Phenyl,
Naphthyl,
2-Methoxyphenyl,
4-Methylphenyl,
4-Bromophenyl,
Benzyl,
2-Phenylethyl,
4-Fluorophenylmethyl,
4-Methoxyphenylmethyl,
3,4,5-Trimethoxyphenylmethyl,
Diphenylmethyl,
Aminocarbonyl,
6-(9-β-D-Ribofuranosid)-adenyl oder
4-Amino-6,7-dimethoxychinazolinyl,
2-Furylcarbonyl, und
n eine ganze Zahl von 3 bis 10 ist, oder ein Hydrochinon-Derivat derselben.

2. Verbindung nach Anspruch 1, worin n 4, 5 oder 6 ist.

3. Verbindung nach Anspruch 1, worin $R^2$ Amino, Carboxymethylamino oder 4-Phenethylpiperidino ist.

4. Verbindung nach Anspruch 1, worin $R^1$ Phenyl oder ein Phenyl mit einem Halogen, Methyl oder Methoxy in einer wahlfreien Position am Phenyl-Ring ist.

5. Verbindung nach Anspruch 1, worin $R^1$ Phenyl oder ein Phenyl mit einem Halogen in der meta- oder para-Position am Phenyl-Ring ist.

6. Verbindung nach Anspruch 1, worin $R^2$ Aralkyl ist, das aus Benzyl, α-Phenethyl, β-Phenethyl und 1-(α-

Naphthyl)ethyl ausgewählt ist.

7. Verbindung nach Anspruch 1, worin $R^2$ der in Anspruch 1 definierte Aminosäure-Rest ist.

8. Verbindung nach Anspruch 1, worin $R^2$ eine aus PiperAzino und Pyrrolidino ausgewählte cyclische Amino-Gruppe ist, wobei die cyclische Amino-Gruppe unsubstituiert oder substituiert ist, wie in Anspruch 1 spezifiziert ist.

9. Verbindung nach Anspruch 1, worin die Verbindung 7-(3,5,6-Trimethyl-1,4-benzochinon-2-yl)-7-phenylheptanamid ist.

10. Verbindung nach Anspruch 1, worin die Verbindung 7-(3,5,6-Trimethyl-1,4-benzochinon-2-yl)-7-phenylheptanoylglycin ist.

11. Verbindung nach Anspruch 1, worin die Verbindung 1-[7-(3,5,6-Trimethyl-1,4-benzochinon-2-yl)-7-phenylheptanoyl]-4-(2-phenylethyl)piperidin ist.

12. Verfahren zur Herstellung eines Chinon-Derivats der allgemeinen Formel (I), wie es in Anspruch 1 definiert ist, oder eines Hydrochinon-Derivats desselben, umfassend :

(i) das Umsetzen eines Carbonsäure-Aktivators mit einer Verbindung der Formel (II)

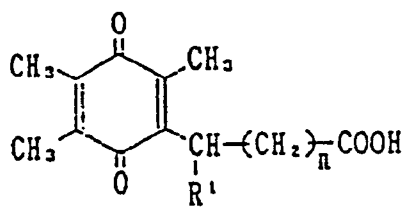

(II)

(worin $R^1$ und n die in Anspruch 1 angegebenen Bedeutungen haben) zur Bildung eines reaktionsfähigen Derivats an der Carboxyl-Gruppe und danach

(ii) das Umsetzen der resultierenden Verbindung mit einer Verbindung der allgemeinen Formel

$R^2$-H

worin $R^2$ die in Anspruch 1 für $R^2$-H angegebenen Bedeutungen hat, und gewünschtenfalls die Umwandlung der Chinon-Verbindung (I) in ein Hydrochinon-Derivat derselben.

13. Pharmazeutische Zusammensetzung, umfassend als wirksame Komponente eine Chinon-Verbindung (I) nach irgendeinem der Ansprüche 1 bis 12 oder ein Hydrochinon-Derivat derselben der allgemeinen Formel (I')

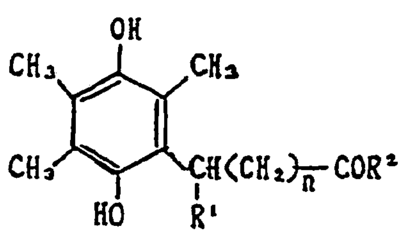

(I')

worin jedes der Symbole die im Vorstehenden angegebenen Bedeutungen hat, zusammen mit einem pharmazeutisch annembaren Träger oder Verdünnungsmittel dafür.

14. Pharmazeutische Zusammensetzung nach Anspruch 13, die ein therapeutisches Mittel für die Behandlung von Asthma oder Arteriosklerose oder ein antiallergisches Mittel ist.

15. Verwendung einer Verbindung (1) nach irgendeinem der Ansprüche 1 bis 12 oder eines Hydrochinon-Derivats derselben zur Herstellung eines therapeutischen Mittels für die Behandlung von Asthma oder Arteriosklerose oder eines antiallergischen Mittels.

**Patentansprüche fur die benannten Vertragsstaaten AT ES**

1. Verwendung einer Verbindung der allgemeinen Formel

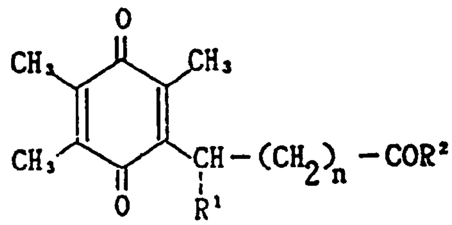

(I)

in der

R [1] unsubstituiertes Phenyl oder Phenyl ist, das in einer wahlfreien Position am Ring mit

Hydroxyl

Halogen,

Alkyl mit 1 bis 3 Kohlenstoff-Atomen oder

Alkoxy mit 1 bis 3 Kohlenstoff-Atomen substituiert ist, BR> $R^2$ unsubstituiertes Amino, Amino, das substituiert ist mit

$(C_{1-4})$-Alkyl,

unsubstituiertem $(C_{6-10})$-Aryl,

$(C_{6-10})$-Aryl, das substituiert ist mit

Hydroxyl,

Amino,

Nitro,

Halogen,

Methyl oder

Methoxy,

Benzyl,

α-Phenethyl,

β-Phenethyl,

1-(α-Naphthylethyl),

einem Aminosäure-Rest, der von der Eliminierung eines Wasserstoffs der Amino-Gruppe einer Aminosäure aus der aus

Glycin,

Arginin,

Histidin,

Asparaginsäure,

Prolin,

Phenylalanin,

Methionin,

Alanin,

Leucin und

Glutaminsäure

bestehenden Gruppe herrührt, wobei der Aminosäure-Rest unverestert oder an der Carboxy-Gruppe mit Benzylalkohol verestert ist oder

der Aminosäure-Rest ungeschützt oder, wenn der Aminosäure-Rest eine Amino-Gruppe besitzt, durch Nitro geschützt ist,

cyclischem Amino, das aus der aus

Morpholino,

Piperidino,

Piperazino oder

Pyrrolidino

bestehenden Gruppe ausgewählt ist, wobei das cyclische Amino unsubstituiert oder substituiert ist mit

Methyl,

Ethyl,

Propyl,

Pyrrolidinocarbonylmethyl,

4-Fluorophenylcarbonylpropyl,

Phenyl,

Naphthyl,

2-Methoxyphenyl,
4-Methylphenyl,
4-Bromophenyl,
Benzyl,
2-Phenylethyl,
4-Fluorophenylmethyl,
4-Methoxyphenylmethyl,
3,4,5-Trimethoxyphenylmethyl,
Diphenylmethyl,
Aminocarbonyl,
6-(9-β-D-Ribofuranosid)-adenyl oder
4-Amino-6,7-dimethoxychinazolinyl,
2-Furylcarbonyl, und
n eine ganze Zahl von 3 bis 10 ist, oder eines Hydrochinon-Derivats derselben zur Herstellung eines therapeutischen Mittels für die Behandlung von Asthma oder Arteriosklerose oder eines antiallergischen Mittels.

2. Verfahren zur Herstellung eines Chinon-Derivats der allgemeinen Formel (I), wie es in Anspruch 1 definiert ist, oder eines Hydrochinon-Derivats desselben, umfassend :

(i) das Umsetzen eines Carbonsäure-Aktivators mit einer Verbindung der Formel (II)

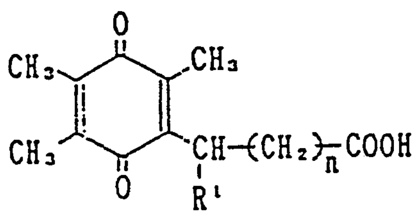
(II)

(worin $R^1$ und n die in Anspruch 1 angegebenen Bedeutungen haben) zur Bildung eines reaktionsfähigen Derivats an der Carboxyl-Gruppe und danach

(ii) das Umsetzen der resultierenden Verbindung mit einer Verbindung der allgemeinen Formel

$R^2$-H

worin $R^2$ die in Anspruch 1 für angegebenen Bedeutungen hat, und gewünschtenfalls die Umwandlung der Chinon-Verbindung (I) in ein HydrochinonDerivat derselben.

3. Verfahren nach Anspruch 2, worin n 4, 5 oder 6 ist.

4. Verfahren nach Anspruch 2, worin $R^2$ Amino, Carboxymethylamino oder 4-Phenethylpiperidino ist.

5. Verfahren nach Anspruch 2, worin $R^1$ Phenyl oder ein Phenyl mit einem Halogen, Methyl oder Methoxy in einer wahlfreien Position am Phenyl-Ring ist.

6. Verfahren nach Anspruch 2, worin $R^1$ Phenyl oder ein Phenyl mit einem Halogen in der meta- oder para-Position am Phenyl-Ring ist.

7. Verfahren nach Anspruch 2, worin $R^2$ Aralkyl ist, das aus Benzyl, α-Phenethyl, β-Phenethyl und 1-(α-Naphthyl)ethyl ausgewählt ist.

8. Verfahren nach Anspruch 2, worin $R^2$ der in Anspruch 1 definierte Aminosäure-Rest ist.

9. Verfahren nach Anspruch 2, worin $R^2$ eine aus Piperazino und Pyrrolidino ausgewählte cyclische Amino-Gruppe ist, wobei die cyclische Amino-Gruppe unsubstituiert oder substituiert ist, wie in Anspruch 1 spezifiziert ist.

10. Verfahren nach Anspruch 2, worin die Verbindung 7-(3,5,6-Trimethyl-1,4-benzochinon-2-yl)-7-phenylheptanamid ist.

11. Verfahren nach Anspruch 2, worin die Verbindung 7-(3,5,6-Trimethyl-1,4-benzochinon-2-yl)-7-phenylheptanoylglycin ist.

12. Verfahren nach Anspruch 2, worin die Verbindung 1-[7-(3,5,6-Trimethyl-1,4-benzochinon-2-yl)-7-phenylheptanoyl]-4-(2-phenylethyl)piperidin ist.

13. Pharmazeutische Zusammensetzung, umfassend als wirksame Komponente eine Chinon-Verbindung (I) nach irgendeinem der Ansprüche 1 bis 12 oder ein Hydrochinon-Derivat derselben der allgemeinen Formel (I')

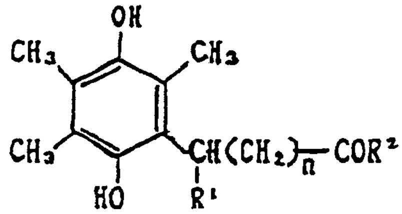

(I ')

worin jedes der Symbole die im Vorstehenden angegebenen Bedeutungen hat, zusammen mit einem pharmazeutisch annembaren Träger oder Verdünnungsmittel dafür.

14. Pharmazeutische Zusammensetzung nach Anspruch 13, die ein therapeutisches Mittel für die Behandlung von Asthma oder Arteriosklerose oder ein antiallergisches Mittel ist.

## Revendications

### Revendications pour les Etats contractants : BE, CH, DE, FR, GB, GR, IT, LI, LU, NL, SE

1. Composé de formule générale :

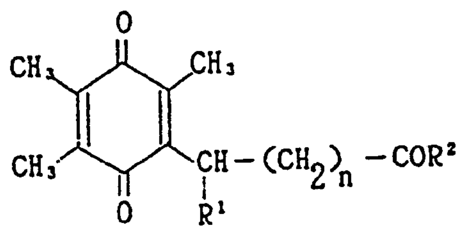

(I)

dans laquelle

$R^1$ est un phényle non substitué ou un phényle substitué en une position quelconque du cycle par un hydroxyle, un halogène, un alcoyle comportant de 1 à 3 atomes de carbone ou un alcoxy comportant de 1 à 3 atomes de carbone,

$R^2$ est un amino non substitué ou un amino substitué par

un alcoyle en $C_{1-4}$,

un aryle en $C_{6-10}$ non substitué,

un aryle en $C_{6-10}$ substitué par un hydroxyle, un amino, un nitro, un halogène, un méthyle ou un méthoxy,

un benzyle,

un α-phénéthyle,

un β-phénéthyle,

un 1-(α-naphtyl)éthyle,

un radical d'un acide aminé formé par élimination d'un hydrogène du groupe amino, consistant en la glycine, l'arginine, l'histidine, l'acide aspartique, la proline, la phénylalanine, la méthionine, l'alanine, la leucine et l'acide glutamique, dont le radical d'acide aminé est non estérifié ou estérifié au groupe carboxylique par l'alcool benzylique ou le radical d'acide aminé est non protégé ou protégé, lorsque le radical d'acide aminé comporte un groupe amino, par un nitro,

un groupe amino cyclique choisi dans le groupe consistant en morpholino, pipéridino, pipérazino ou pyrrolidino,

le groupe amino cyclique étant non substitué ou substitué par un méthyle, éthyle, propyle, pyrrolidinocarbonylméthyle, 4-fluorophénylcarbonylpropyle, phényle, naphtyle, 2-méthoxyphényle, 4-méthylphényle, 4-bromophényle, benzyle, 2-phényléthyle, 4-fluorophénylméthyle, 4-méthoxyphénylméthyle, 3,4,5-triméthoxyphénylméthyle, diphénylméthyle, aminocarbonyle, 6-(9-β-D-ribofuranoside)-adényle ou 4-amino-6,7-diméthoxy-quinazolinyle, 2-furylcarbonyle

et un est un nombre entier de 3 à 10, ou un dérivé hydroquinonique de ce composé.

2. Composé selon la revendication 1, dans lequel n est 4, 5 ou 6.

3. Composé selon la revendication 1, dans lequel $R^2$ est un groupe amino, carboxyméthylamino ou 4-phénéthylpipéridino.

4. Composé selon la revendication 1, dans lequel $R^1$ est un groupe phényle ou phényle comportant un halo-

gène, un méthyle ou un méthoxy en une position quelconque du cycle phénylique.

5. Composé selon la revendication 1, dans lequel $R^1$ est un groupe phényle ou phényle comportant un halogène en la position méta ou para du groupe phénylique.

6. Composé selon la revendication 1, dans lequel $R^2$ est un groupe aralcoyle choisi parmi les groupes benzyle, α-phénéthyle, β-phénéthyle et 1-(α-naphtyl)éthyle.

7. Composé selon la revendication 1, dans lequel $R^2$ est ledit radical d'un acide aminé tel que défini à la revendication 1.

8. Composé selon la revendication 1, dans lequel $R^2$ est un groupe amino cyclique choisi parmi les groupes pipérazino et pyrrolidino, ledit groupe amino cyclique étant non substitué ou substitué comme défini à la revendication 1.

9. Composé selon la revendication 1, qui est le 7-(3,5,6-triméthyl-1,4-benzoquinone-2-yl)-7-phénylheptanamide.

10. Composé selon la revendication 1, qui est la 7-(3,5,6-triméthyl-1, 4-benzoquinone-2-yl)-7-phénylheptanoylglycine.

11. Composé selon la revendication 1, qui est la 1-[7-(3,5,6-triméthyl-1, 4-benzoquinone-2-yl)-7-phénylheptanoyl]-4-(2-phényléthyl)pipéridine.

12. Procédé de préparation d'un dérivé quinonique de formule générale (I) tel que défini à la revendication 1 ou d'un dérivé hydroquinonique de celui-ci, selon lequel :

(i) on fait réagir un activateur d'acide carboxylique avec un composé de formule générale (II) :

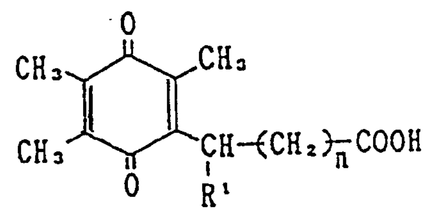

(II)

dans laquelle $R^1$ et n sont tels que défini à la revendication 1, pour former un dérivé réactif du groupe carboxylique ; puis,

(ii) on fait réagir le composé obtenu avec un composé de formule générale :

$$R^2\text{-}H$$

dans laquelle $R^2$ est tel que défini à la revendication 1, et si désiré, on transforme ledit composé quinonique (I) en un dérivé hydroquinonique de celui-ci.

13. Composition pharmaceutique, qui comprend comme substance active un composé quinonique (I) tel que défini à l'une quelconque des revendications 1 à 12, ou un dérivé hydroquinonique de ce composé, de formule générale (I') :

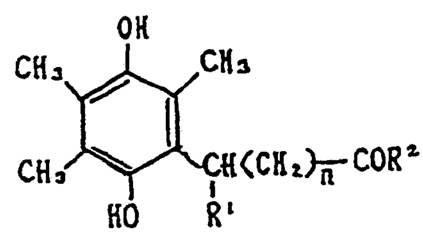

(I')

dans laquelle chacun des symboles est tel que défini ci-dessus, ensemble avec un véhicule ou diluant pharmaceutiquement acceptable.

14. Composition pharmaceutique selon la revendication 13, qui est un agent thérapeutique pour le traitement de l'asthme ou de l'artériosclérose ou un agent antiallergique.

15. Utilisation du composé (I) tel que défini à l'une quelconque des revendications 1 à 12 ou d'un dérivé hydroquinonique de celui-ci, pour la préparation d'un agent thérapeutique pour le traitement de l'asthme ou de l'artériosclérose ou d'un agent antiallergique.

**Revendications pour les Etats contractants : AT ES**

1. Utilisation, pour la préparation d'un agent thérapeutique pour le traitement de l'asthme ou de l'artériosclérose ou d'un agent antiallergique, d'un composé quinonique de formule générale (I) :

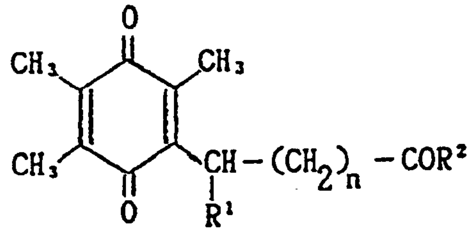

(I)

dans laquelle
$R^1$ est un phényle non substitué ou un phényle substitué en une position quelconque du cycle par un hydroxyle, un halogène, un alcoyle comportant de 1 à 3 atomes de carbone ou un alcoxy comportant de 1 à 3 atomes de carbone,
$R^2$ est un amino non substitué ou un amino substitué par
un alcoyle en $C_{1-4}$,
un aryle en $C_{6-10}$ non substitué,
un aryle en $C_{6-10}$ substitué par un hydroxyle, un amino, un nitro, un halogène, un méthyle ou un méthoxy,
un benzyle,
un α-phénéthyle,
un-β-phénéthyle,
un 1-(α-naphtyl)éthyle,
un radical d'un acide aminé formé par élimination d'un hydrogène du groupe amino, consistant en la glycine, l'arginine, l'histidine, l'acide aspartique, la proline, la phénylalanine, la méthionine, l'alanine, la leucine et l'acide glutamique, dont le radical d'acide aminé est non estérifié ou estérifié au groupe carboxylique par l'alcool benzylique ou le radical d'acide aminé est non protégé ou protégé, lorsque le radical d'acide aminé, comporte un groupe amino, par un nitro,
un groupe amino cyclique choisi dans le groupe consistant en morpholino, pipéridino, pipérazino ou pyrrolidino, le groupe amino cyclique étant non substitué ou substitué par un méthyle, éthyle, propyle, pyrrolidinocarbonylméthyle, 4-fluorophénylcarbonylpropyle, phényle, naphtyle, 2-méthoxyphényle, 4-méthylphényle, 4-bromophényle, benzyle, 2-phényléthyle, 4-fluorophénylméthyle, 4-méthoxyphénylméthyle, 3,4,5-triméthoxyphénylméthy, diphénylméthyle, aminocarbonyle, 6-(9-β-D-ribofuranoside)-adényle ou 4-amino-6,7-diméthoxy-quinazolinyle, 2-furylcarbonyle et n est un nombre entier de 3 à 10 ou un dérivé hydroquinonique de ce composé.

2. Procédé de préparation d'un dérivé quinonique de formule générale (I) tel que défini à la revendication 1 ou d'un dérivé hydroquinonique de celui-ci, selon lequel :
(i) on fait réagir un activateur d'acide carboxylique avec un composé de formule générale (II) :

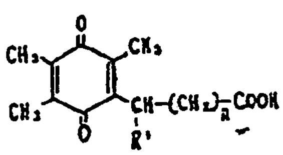

(II)

dans laquelle $R^1$ et n sont tels que défini à la revendication 1, pour former un dérivé réactif du groupe carboxylique ; puis,
(ii) on fait réagir le composé obtenu avec un composé de formule générale :

$$R^2\text{-}H$$

dans laquelle $R^2$ est tel que défini à la revendication 1, et si désiré, on transforme ledit composé quinonique (I) en un dérivé hydroquinonique de celui-ci.

3. Procédé selon la revendication 2, dans lequel n est 4, 5 ou 6.

4. Procédé selon la revendication 2, dans lequel $R^2$ est un groupe amino, carboxyméthylamino ou 4-phé-

néthylpipéridino.

5. Procédé selon la revendication 2, dans lequel $R^1$ est un groupe phényle ou phényle comportant un halogène, un méthyle ou un méthoxy en une position quelconque du cycle phénylique.

6. Procédé selon la revendication 2, dans lequel $R^1$ est un groupe phényle ou phényle comportant un halogène en la position méta ou para du groupe phénylique.

7. Procédé selon la revendication 2, dans lequel $R^2$ est un groupe aralcoyle choisi parmi les groupes benzyle, α-phénéthyle, β-phénéthyle et 1-(α-naphtyl)éthyle.

8. Procédé selon la revendication 2, dans lequel $R^2$ est ledit radical d'un acide aminé tel que défini à la revendication 1.

9. Procédé selon la revendication 2, dans lequel $R^2$ est un groupe amino cyclique choisi parmi les groupes pipérazino et pyrrolidino, ledit groupe amino cyclique étant non substitué ou substitué comme défini à la revendication 1.

10. Procédé selon la revendication 2, dans lequel le composé est le 7-(3,5,6-triméthyl-1,4-benzoquinone-2-yl)-7-phénylheptanamide.

11. Procédé selon la revendication 2, dans lequel le composé est la 7-(3,5,6-triméthyl-1,4-benzoquinone-2-yl)-7-phénylheptanoylglycine.

12. Procédé selon la revendication 2, dans lequel le composé est la 1-[7-(3,5,6-triméthyl-1,4-benzoquinone-2-yl)-7-phénylheptanoyl]-4 -(2-phényléthyl)pipéridine.

13. Composition pharmaceutique, qui comprend comme substance active un composé quinonique (I) tel que défini à l'une quelconque des revendications 1 à 12, ou un dérivé hydroquinonique de ce composé, de formule générale (I') :

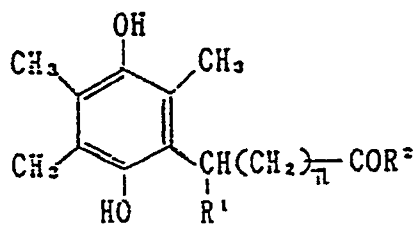

(I')

dans laquelle chacun des symboles est tel que défini ci-dessus, ensemble avec un véhicule ou diluant pharmaceutiquement acceptable.

14. Composition pharmaceutique selon la revendication 13, qui est un agent thérapeutique pour le traitement de l'asthme ou de l'artériosclérose ou un agent antiallergique.